# EUROPEAN PATENT APPLICATION

(11) **EP 4 104 804 A1**
(43) Date of publication of application: **21.12.2022**
(21) Application number: 21180154.3
(22) Date of filing: 17.06.2021
(51) Int. Cl.: A61F 13/10

(54) **A FINGER BANDAGE AID AND A PROCESS FOR THE PROVISION OF SAID FINGER BAND-AGE AID**

(71) Applicant: Universität Zürich, 8006 Zürich (CH)
(72) Inventor: GUIDI, Marco, 8050 Zürich (CH); ZUBERBÜHLER, Aldo, 8057 Zürich (CH); KALLEN, Manuel, 8050 Zürich (CH)
(74) Representative: Kleine, Hubertus

(57) **Abstract**

A Finger bandage aid (1) comprising a handle (2) for holding said aid (1) and a number of arms (4, 5, 6) for the spreading of a tubular-formed or sock-formed bandage (18), such that at least one or multiple fingers (19) or a hand can be introduced in a space (13) between the arms (4, 5, 6) by application of said bandage (18) around said finger (19), wherein the finger bandage aid (1) comprises an actuation device and a channel (7) at a transition area (3) between said handle (2) and said arms (4, 5, 6), wherein at least a part of said actuation device is arranged movably inside the channel (7) and wherein the distance between said arms (4, 5, 6) is changeable by a movement of said actuation device in the channel (7).

## Description

### Technical Field

The subject of the present invention relates to a finger bandage aid according to the preamble of claim 1.

A further subject is a process for the provision of an individually adapted finger bandage aid, for the specific size of a finger.

### Background Art

FR 1 215 718 A discloses in Fig. 4 a finger bandage aid with a handle section and multiple arms in the form of spokes. Said arms are guided very close to the finger during the slipping of the bandage over the finger. The adjustment of the space between the arms bears the risk of breaking the arms during their adjustment. Even in the case that the arms might be slightly adjusted to the form and/or size of a finger, said adjustment will be done at the arms and not in the transition area between the arms and the handle, where the risk of a break is the highest in this device. Due to the tapering of the arms towards the transition area, the bandage operation may cause pain depending on the level of injuries inside the finger.

### Object of the invention

With regard to the cited state-of-the-art it is the object of the current invention to provide a finger bandage aid which is better adaptable to an individual form and size of a finger, multiple fingers or a whole hand and therefore causes less pain during the bandage operation.

### Disclosure of the invention

The current invention overcomes the aforementioned problems resulting from said state-of-the-art with the subject-matter of claim 1.

An inventive finger bandage aid comprises a handle for holding said aid and a number of arms for the spreading of a tubular-formed or sock-formed bandage, such that a finger, multiple fingers or even a hand can be introduced in a space between said arms. During said introduction procedure said bandage is applied around said finger, fingers or hand.

The finger bandage aid is therefore not reduced to a bondage operation of a single finger but can also be used for the bandage of multiple fingers or even the whole hand.

The finger bandage aid comprises an actuation device, preferably in the form of a locking pin, and a channel at a transition area between said handle and said arms, wherein at least a part of said actuation device is arranged movably inside the channel and wherein the distance between said arms is changeable and therefore adjustable by a movement of said actuation device in the channel.

The transition area comprises an Intersection area of the arms from which the arms extend from the handle and where the arms are connected to each other.

During the application of the bandage the arms should remain at the position and should therefore be stable but during the adaptation before the application of the bandage the arms should be adaptable the facilitate the pull-over of the bandage. The distance between the arms could easily be adapted by moving said actuation device.

By moving the locking pin the arms may be moved at the position of the transition area, so that this area preferably has the function of a hinge. After the movement of said pin in a locking position the arms should remain at the adapted predefined distance to each other.

The form of the space defined by the arms may be described as aval-shaped, preferably pointed oval at one side of said space.

Due to the possibility of an adaptation at the transition area the inventive finger band aid of the current invention generates less pain due to the possibility of an individual adaptation of the aid to the form and size of a finger, a number of fingers or a hand to be bandaged.

Further advantageous embodiments of the invention may be described in the context of the subject matter of the dependent claims.

The actuation device can be a locking pin. Said the locking pin can preferably be provided with a pin head and a shaft. This way the channel can be compared to a dowel which is spread by a screw.

Said locking pin can be arranged movably inside said channel. Said movement is preferably linear to the axis of the channel but may include a linear or rotational movement of the pin.

The inner wall of the transition area limiting the channel can have a conical shape to enhance the spreading of the channel and therefore the arms.

The channel, preferably the inner wall, can be provided with latching grooves for a stepwise adjustment and/or with a thread for continuous adjustment of the position of the pin head inside the channel.

The transition area around the channel can be provided with one or more slits. The slit or the slits is or are more preferably opened at one end of the respective slit.

The slit or the slits can preferably extend parallel to the longitudinal axis of the channel.

The width of the slit or the slits preferably depends on the position of the locking pin in the channel.

For a better introduction of the finger an arm and more preferably each arm preferably has an arcuated form.

In a preferred embodiment of the invention each arm might have a rectangular or U-shaped cross section. A rectangular form in the context of the current invention may comprise rounded edges and slightly bended sides.

The arms may have uniform shape and could be uniformly spaced apart from each other.

Each of said arms could be provided with a rounded distal end, preferably a spherical or ovoidal distal end. This reduces the risk of damages of the bandage material.

The finger bandage aid might have a longitudinal axis which is coaxial with the direction of introduction of the finger in the space defined by the arm, wherein the radial thickness of said arms in relation to said longitudinal axis is higher than 0.2 cm, preferably between 0.3-0.8 cm.

The thickness of the arms perpendicular to said radial thickness could be at least 150%, preferably at least 200 %, more preferably 250-500% of said radial thickness.

The extension of the handle in axial direction of said longitudinal axis could preferably be less than 40% of the extension of the arms in the axial direction.

The actuation element could preferably protrude over the end of the handle, such that the distal distance between the arms is adjustable by actuation of the part of the actuation element which protrudes over the end of the handle.

A further aspect of the current invention is the use of a previously described finger bandage aid for the application of a bandage at one or more fingers or a whole hand by slipping over said finger, fingers or hand.

A further aspect of the invention is a process for the provision of a bandage at an inventive finger bandage aid comprising the following steps:

| | |
|---|---|
| A | Adjusting the space (18) between the arms (4, 5, 6) by moving the actuation element to a first position inside the channel (7); |
| B | Application of the bandage over said arms (4, 5, 6); and |
| C | Widening the space (18) between the arms (4, 5, 6) by moving the actuation element to a second position inside the channel (7); such that the finger bandage aid is provided to be applied to a finger. |

A bandage provided by the finger bandage aid forms a flexible soft stop for the formation of a bandage layer at the tip of the finger, fingers or hand during the introduction of the finger, fingers or hand inside the space between said arms.

Said inventive process may preferably comprise further steps. One further step may be the introduction of a finger, fingers or a hand inside said space.

Another further step may be the pulling away of the finger bandage aid from said finger while the bandage partly remains at said finger.

Another further step might be the twisting of the handle to twist the bandage at the top of said finger.

Another further step may be the pushing the finger bandage aid towards said finger while the bandage partly remains at said finger (and covers the first layer of bandage).

Another further step may be the repeating of the aforementioned 3 steps several times.

The aforementioned further steps however may be known from the cited state of the art.

### Brief Description of the drawings (Figures and Tables)

An advantageous embodiment of a finger bandage aid is further explained in detail below together with drawings. Specific parts of the embodiment can be understood as separate features that can also be realized in other embodiments of the invention. The combination of features described by the embodiment shall not be understood as a limitation for the invention:
- Fig. 1: a perspective view of an embodiment of the inventive finger bandage aid; and
- Fig. 2: a perspective cross-sectional view of the finger bandage aid of Fig. 1.

### Mode for Carrying out the invention

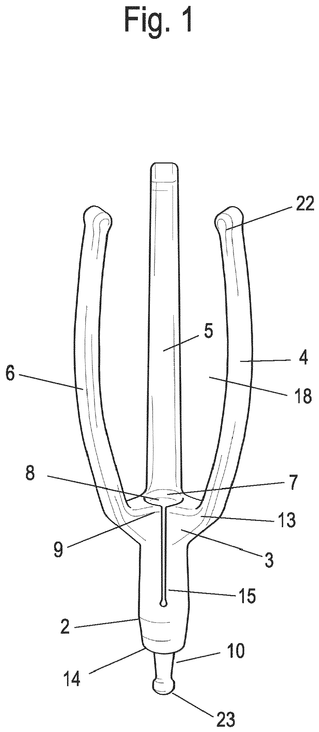

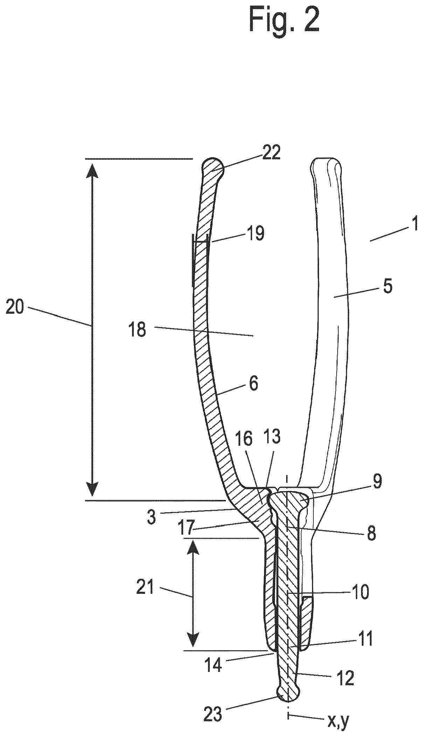

An embodiment of an inventive finger bandage aid 1 is shown in **Fig. 1** and **Fig. 2****.** The finger bandage aid 1 comprises a handle 2 having a longitudinal axis X, wherein said handle 2 can be gripped with two or more fingers during the application of the bandage to a finger with the help of said bandage aid 1.

The finger bandage aid 1 is provided with three arms 4, 5 and 6 extending from said handle 2, thus the structure of the finger bandage aid 1 can be described as tripodal. The area from which the arms 4-6 are extending is called transition area 3. Said arms 4-6 have each an arcuated form and are basically identical and are equally distanced defining an angle α of 120° from the center axis of each arm 4-6. The arms 4-6 have each a rounded distal end, most preferably a spherical or ovoidal distal end, such that the end has partly the form of a sphere or an ovoid.

The transition area 3 is provided with a channel 7 parallel or preferably coaxial with the longitudinal axis X of the handle 2. As shown in Fig. 1 the channel 7 may extend over the whole length of the handle.

Inside the channel 7 a locking pin 8 is provided. The locking pin 8 is movable along the axis Y, defined by the channel. The movement of the locking pin 8 might preferably be a translational movement or a rotational movement. Said rotational movement can be a screwing movement, such that the locking pin moves along the axis Y during the rotation.

The locking pin 8 can be provided with a pin head 9 and a shaft 10, wherein the diameter of the pin head 9 is larger than the diameter of at least a first part 11 the shaft 10 which is positioned inside the channel 7.

The pin head 9 is located inside the channel 7. In a preferred embodiment a second part 12 of the shaft 10 protrudes over the end of the handle 2, such that the locking pin 8 can be moved by actuation of the second part 12 of the locking pin 8. Said second part 12 can be provided with a knob 23 at a distal end of said second part to facilitate rotation of the handle.

Doing so, the channel 7 may grow or diminish in diameter at one first end 13 of the channel 7. Said first end 13 is opposite to a second end 14 of the channel 7 which is at the end of the handle 2, where the second part 12 of the shaft 10 protrudes from the handle 2.

For the variation of the diameter of the channel 7 at said first end 13 the wall, which defines the channel, is provided with slits 15. In the case of the embodiment shown in Fig. 1 said slits 15 extend parallel to the axis Y of the channel 7 from the transition area 3 to the handle 2.

The slits 15 can be widened by the introducing the pin head 9 into the channel 7. In a preferred embodiment of the invention the channel 7 can be provided in an area next to said first end 13 with a conical inner wall 16 wherein the diameter reduces from the end 13 towards the middle of channel 7 and thus the middle of the handle 2.

The slits 15 are provided over at least 30%, preferably between 40-75 % of the length of the handle.

The conical inner wall 16 might have internal locking positions wherein ring-shaped latching grooves 17 that can partly form a form-fit with the pin head 9 at a spreading position of the locking pin 8.

The arms 4-6 define an oval space 18 with at least one pointed end towards the transition area 3. The transition area 3 comprises an intersection point 11 where the arcuated center axis of the three arms 4-6 cross each other.

The longitudinal axis X may extend though the space 18. The radial thickness 19 of each arm is in the current example about 0.5 cm. The thickness perpendicular to the said radial thickness is about 2 cm.

The axial extension 20 of each arm 4-6 along the longitudinal axis 14 is less than 13 cm and the axial extension 21 of the handle 2 is less than 4 cm.

The finger bandage aid 1 might have a preferred length of 12-15 cm.

The handle 2 might by further provided with a corrugation or a fluting or the like for a better grip during the use of the aid 1. A preferred diameter of the handle is between 0.5 - 3 cm.

The diameter of the inner circle of the three arms can be adopted by moving the locking pin preferably between 4,5-7 cm.

The arms 4-6 are connected to the transition area 3 in a firmly bonded manner. Preferably the material of the arms 4-6, the transition area 3 and the handle consist of the same material.

The connection zone of the arms 4-6 to the transition area 3 is positioned between two slits 15, such that if the slits are widened or reduced due to a movement of the locking pin 8 within the channel 7, the distance between the arms 4-6 will be widened or deduced likewise. Preferably three slits 15 are provided in the finger bandage aid 1.

As mentioned already before, the wherein ring-shaped latching grooves 17 can be replaced by a thread, While the position of the pin head 9 is discrete and depend on the axial position of said ring-shaped latching grooves 17, the position of the pin head 9 can be adopted to be continuous due to the use of a thread.

The finger bandage aid can be provided by only two pieces, one first integrally-formed piece provided with the arms 4-6, the handle 2 and the transition area 3, including the channel 7 and a second piece which is the locking pin 8 that can be moved inside the channel relative to said first piece and thus change the distance of the arms 4-6. The spreading of the arms 4-6 is reversible, due to the flexible plastic material. The length of the slits 15 may depend on the flexibility of the plastic material that is used. A suitable plastic material can be a polyolefin such as Polypropylene and/or Polyethylene with or without plasticizer for an adaptation of a suitable flexibility.

The arms 4-6 can be provided with a round shape, preferably with egg or ball-shaped forms 22, at their distal ends to reduce the risk of friction and damage of the gauze positioned external to the arms 4-6-

The finger bandage aid is provided to prevent misuse. It can be used without any further description in an intuitive way.

The finger bandage aid preferably has a tripodal form. A symmetry of the arm-deformation is achieved by the technical design disclosed by the embodiment of Fig. 1 and 2

At least the first integrally-formed piece can be a plastic material and can be manufactured by injection molding. The second piece can also preferably be a plastic material or a metal material.

An advantage of the aforementioned finger bandage aid is the avoidance of an overstretching of a finger.

A further advantage might be that the construction of the finger bandage aid can be achieved by numerous plastic materials.

### List of references

- 1: finger bandage aid
- 2: handle
- 3: transition area
- 4: arm
- 5: arm
- 6: arm
- 7: channel
- 8: locking pin
- 9: pin head
- 10: shaft
- 11: first part
- 12: second part
- 13: first end
- 14: second end
- 15: slit
- 16: inner wall
- 17: latching grooves
- 18: oval space
- 19: radial thickness
- 20: axial extension (arm)
- 21: axial extension (handle)
- 22: ball shaped form
- 23: knob

- X: longitudinal axis (handle)
- Y: longitudinal axis (channel)

## Claims

1. Finger bandage aid (1) comprising a handle (2) for holding said aid (1) and a number of arms (4, 5, 6) for the spreading of a tubular-formed or sock-formed bandage, such that at least one or multiple fingers or a hand can be introduced in a space (13) between the arms (4, 5, 6) by application of said bandage around said finger, **characterized in that** the finger bandage aid (1) comprises an actuation device and a channel (7) at a transition area (3) between said handle (2) and said arms (4, 5, 6), wherein at least a part of said actuation device is arranged movably inside the channel (7) and wherein the distance between said arms (4, 5, 6) is changeable by a movement of said actuation device in the channel (7).

2. Finger bandage aid according to claim 1, **characterized in that** actuation device is a locking pin (8), wherein the locking pin (8) is preferably provided with a pin head (9) and a shaft (10).

3. Finger bandage aid according claim 2, **characterized in that** said locking pin (8) is arranged movably inside said channel (7).

4. Finger bandage aid according to one of the aforementioned claims, **characterized in that** the inner wall (16) of the transition area (3) limiting the channel (7) has a conical shape.

5. Finger bandage aid according to one of the aforementioned claims, **characterized in that** the channel (7), preferably the inner wall, is provided with latching grooves and/or a thread for adjusting the position of the pin head (9) inside the channel (7).

6. Finger bandage aid according to one of the aforementioned claims, **characterized in that** the transition area (3) is provided with one or more slits (15), wherein the slit (15) or the slits (15) is or are preferably opened at one end of the slit (15).

7. Finger bandage aid according to claim 6, **characterized in that** the slit (15) or the slits (15) extend parallel to the longitudinal axis (Y) of the channel (7).

8. Finger bandage aid according to one of the aforementioned claims 6 or 7, **characterized in that** the width of the slit (15) or the slits (15) depend on the position of the locking pin (8) in the channel (7).

9. Finger bandage aid according to one of the aforementioned claims, **characterized in that** the arms (4, 5, 6) have an arcuated form.

10. Finger bandage aid according to one of the aforementioned claims, **characterized in that** each arm (4, 5, 6) has a rectangular or U-shaped cross section.

11. Finger bandage aid according to one of the aforementioned claims, **characterized in that** the arms (4, 5, 6) have uniform shape and are uniformly spaced apart from each other.

12. Finger bandage aid according to one of the aforementioned claims, **characterized in that** each arm (4, 5, 6) is provided with a rounded distal end, preferably a spherical or ovoidal distal end (22).

13. Finger bandage aid according to one of the aforementioned claims, **characterized in that** the actuation element protrudes over the end of the handle (2), such that the distal distance between the arms is adjustable by actuation of part (12) of the actuation element which protrudes over the end of the handle (2).

14. Finger bandage aid according to one of the aforementioned claims, **characterized in that** the finger bandage aid (1) comprises two pieces only.

15. Process for the provision of a bandage at a finger bandage aid (1) according to one of the aforementioned claims comprising the following steps:
A Adjusting the space (18) between the arms (4, 5, 6) by moving the actuation element to a first position inside the channel (7);
B Application of the bandage over said arms (4, 5, 6); and
C Widening the space (18) between the arms (4, 5, 6) by moving the actuation element to a second position inside the channel (7); such that the finger bandage aid is provided to be applied to a finger.
